(19) **Europäisches Patentamt / European Patent Office / Office européen des brevets**

(11) **EP 2 644 282 B1**

(12) **EUROPEAN PATENT SPECIFICATION**

(45) Date of publication and mention
of the grant of the patent:
**21.10.2015 Bulletin 2015/43**

(51) Int Cl.:
**B05B 17/06** (2006.01)     **B05B 17/00** (2006.01)

(21) Application number: **13161185.7**

(22) Date of filing: **26.03.2013**

(54) **Mesh for nebulizer and production method thereof**

Sieb-Zerstäuber und Herstellungsverfahren dafür

Tamis pour nébuliseur et méthod de production de celui-ci

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.03.2012 JP 2012074060**
**25.10.2012 JP 2012235493**

(43) Date of publication of application:
**02.10.2013 Bulletin 2013/40**

(73) Proprietor: **Tanaka Kikinzoku Kogyo K.K.
Chiyoda-ku
Tokyo 100-6422 (JP)**

(72) Inventors:
• **Omura, Yoshinori**
  **KANAGAWA, Kanagawa (JP)**
• **Okazaki, Tsuyoshi**
  **KANAGAWA, Kanagawa (JP)**

(74) Representative: **Gauer, Pierre et al
Feray Lenne Conseil
Le Centralis
63, avenue du Général Leclerc
92340 Bourg-la-Reine (FR)**

(56) References cited:
**EP-A1- 0 516 565     EP-A1- 1 295 647
JP-A- H09 323 054     US-A- 6 085 740**

## Description

[Technical Field]

The present invention relates to a mesh for a nebulizer for use in treatment of respiratory system diseases and the like and to a production method for the mesh, according to claim 1 or 4 respectively.
More particularly, the present invention relates to a mesh for a nebulizer for atomizing liquid and nebulizing the atomized liquid, in which the mesh is used for atomizing liquid and controlling the diameters of particles and has plural through holes.

[Background Art]

In order to enhance the effects of treatment, nebulizers for use in treatment of respiratory system diseases are required to have capability to efficiently make a drug solution reach a target affected area. Decreasing the diameter of particles of a drug solution to be nebulized enables the drug solution to bronchi, bronchioles located back thereof, and alveoli located further back thereof. In addition, the effects of treatment can be enhanced by ensuring a sufficient amount of the nebulized drug solution. Accordingly, in order to improve the capability of the nebulizer, it is necessary to decrease the diameter of particles of the drug solution which is nebulized from the nebulizer, and to increase the amount of the nebulized drug solution.
For example, parabolic-shapes have been proposed as the cross-sectional shapes of through holes in a mesh for a nebulizer (see Non-patent Document 1). Consequently, the fluid resistance of the drug solution can be reduced. Thus, nebulizing efficiency is enhanced. Meanwhile, a cross-sectionally tapered or step-wise shape which is reduced in width towards a central portion in a thickness direction of the mesh nozzle from each of both surfaces thereof (see Patent Document 1). Consequently, even if a through hole is clogged with foreign substance, the foreign substance is blown off by using the nebulizer mesh by flipping the mesh upside down. Thus, the clog can easily be cleared.
Electroforming has mainly been used for processing holes in a nebulizer mesh (see Patent Document 2). In addition, Patent Document 3 and Non-patent Document 2 disclose methods for processing mesh holes by laser irradiation in order to make the cross-sectional shape of each hole to be a stair-like one or a smoothly-tapered one.
Patent Document 4 discloses a mesh nozzle for a nebulizer, which has tapered-shaped through holes wherein a taper angle is equal to or larger than 40 degrees.
Patent Document 4 also discloses that an amount of nebulized liquid per one through hole can be increased by setting the taper angle of through holes as to be equal to or larger than 40 degrees.
Heretofore, palladium-nickel alloy has been used as the materials of the metal meshes (see Patent Document 5). Patent Document 6 discloses an ultrasonic atomizing vibration plate formed of a nickel plate whose surfaces are covered with a thin film made of a metal such as gold, platinum, palladium, or rhodium, or the like.
Additionally, since the nebulizer having a mesh is a medical device, a beauty-treatment device, or the like and the mesh makes direct contact with liquid such as a drug solution or a cosmetic material, it is preferable from a hygienic viewpoint that the mesh is maintained clean by being replaced after a short time of use. Accordingly, in order to enable the short-term replacement of the mesh, the price reduction of the mesh has also become a problem to solve. Thus, resins such as ceramics, whose price is reducible, are also used as the material of the nebulizer mesh (see Non-patent Document 1 and Patent Document 1).

[Prior Art Document]

[Patent Document]

[Patent Document 1] Japanese Patent No. 2,790,014
[Patent Document 2] JP-A-9-323054, which is considered to be the closest prior art and discloses the preamble of claim 1.
[Patent Document 3] JP-A-7-1172
[Patent Document 4] JP-A-2006-297226
[Patent Document 5] JP-A-2008-289903
[Patent Document 6] JP-A-6-320084

[Non-patent Document]

[Non-patent Document 1] OMRON TECHNICS, Vol. 42, pp. 171-175, 2002.
[Non-patent Document 2] The Transactions E of the Institute of Electrical Engineers of Japan, Vol. 117, No. 1, pp. 15-19, 1997.

[Disclosure of the Invention]

[Problems to be Solved by the Invention]

The above conventional shapes of the mesh are not proposed as a result of studying in detail the optimum shape for reducing a nebulized-particle diameter and enhancing an amount of nebulized particles.

Patent Document 3 describes that the amount of nebulized liquid per one through hole can be increased by setting the taper angle of the through hole to be equal to or larger than 40 degrees. However, there is found a problem that if the taper angle is increased, the diameter of particles to be nebulized is increased while the effects of treatment are reduced.

In order to decrease the diameter of nebulized particles, reducing the outlet diameter of the mesh nozzle has been said to be effective. However, the discharge resistance of liquid such as a drug solution is increased only by decreasing the outlet diameter. Thus, there is a risk that the amount of nebulized liquid is reduced and that the effects of the liquid such as the effects of treatment may be reduced as a whole.

Reducing the thickness of the mesh is taken as measures to reduce the discharge resistance. However, thin meshes tend to be insufficient in stiffness. If the stiffness is insufficient, the mesh itself bends. Thus, sufficient pressure cannot be applied to the liquid, such as the drug solution, with which each hole in the mesh is filled. Consequently, the amount of nebulized liquid is decreased.

Additionally, the development of the mesh is not performed by focusing attention to the material of the mesh. For example, the meshes which are respectively described by Non-patent Document 1 and Patent Document 1 are made of ceramics and deficient in stiffness, in comparison with generally-used metal mesh nozzles.

Heretofore, a palladium-nickel alloy or nickel has been used as the material of the metal mesh nozzle, as disclosed in Patent Documents 4 and 5. However, Ni is one of metals which are likely to cause metal allergy. In addition, a subsidiary organization of the World Health Organization (WHO): IARC classifies nickel compounds as "Group 1: carcinogenic compounds to humans". Therefore, there is a problem with safety.

Accordingly, a problem to be solved by the present invention is to provide a nebulizer mesh which is utilizable for treatment of respiratory system diseases or the like, which is capable of setting an average particle diameter (hereinafter referred to as a nebulized -particle diameter) of particles to be nebulized to an optimum particle diameter of particles nebulized from liquid such as a drug solution that reaches target organs such as alveoli; increasing an amount of nebulized particles to thereby enhance the effects of treatment; and ensuring safety and stiffness.

[Means for Solving Problems]

As a result of intensive studies conducted to solve the above problem, the inventors of the present invention have found that the mesh can reduce the nebulized-particle diameter and ensure the sufficient amount of nebulized particles and the sufficient stiffness by employing a tapered shape as the shape of each through hole formed in the nebulizer mesh; setting the taper angle to be within a specific range; and using platinum as a main component to accomplish the present invention.

Namely, the present invention relates to follows:

1. A mesh for use in a nebulizer for atomizing and nebulizing liquid,
wherein the mesh is used to nebulize the liquid, and configured to have a plurality of through holes;
wherein each of the plurality of through holes has a tapered shape whose width is gradually narrowed towards an outlet surface side of the mesh;
wherein each of the plurality of through holes has a taper angle at the outlet surface side ranges from 3 degrees to 30 degrees; and
wherein platinum is used as a main component of a material of the mesh;
2. The mesh for use in a nebulizer according to the above item 1, wherein a thickness of the mesh is equal to or larger than 10 $\mu$m;
3. The mesh for use in a nebulizer according to the above item 1 or 2, wherein an outlet surface side diameter of each of the through holes is equal to or larger than 1.5 $\mu$m;
4. The mesh for use in a nebulizer according to the above items 1 to 3, wherein a particle diameter of nebulized particles from the mesh ranges from 3 $\mu$m to 6 $\mu$m;
5. A method for producing a mesh for use in a nebulizer according to the above item 1, wherein a flat-plat-like member which has platinum as a main component is irradiated with laser light in order to form a plurality of through holes, each of which is cross-sectionally tapered shaped; and

6. The method for producing a mesh for use in a nebulizer according to the above item 5, wherein the laser light is ultraviolet laser light.

[Effects of the Invention]

According to a nebulizer mesh of the present invention, the nebulized-particle diameter can be set to be an optimum particle diameter of particles nebulized from liquid such as a drug solution that reaches target organs such as alveoli. In addition, a sufficient amount of nebulized particles and sufficient stiffness can be ensured.

[Brief Description of the Drawings]

[FIG. 1]

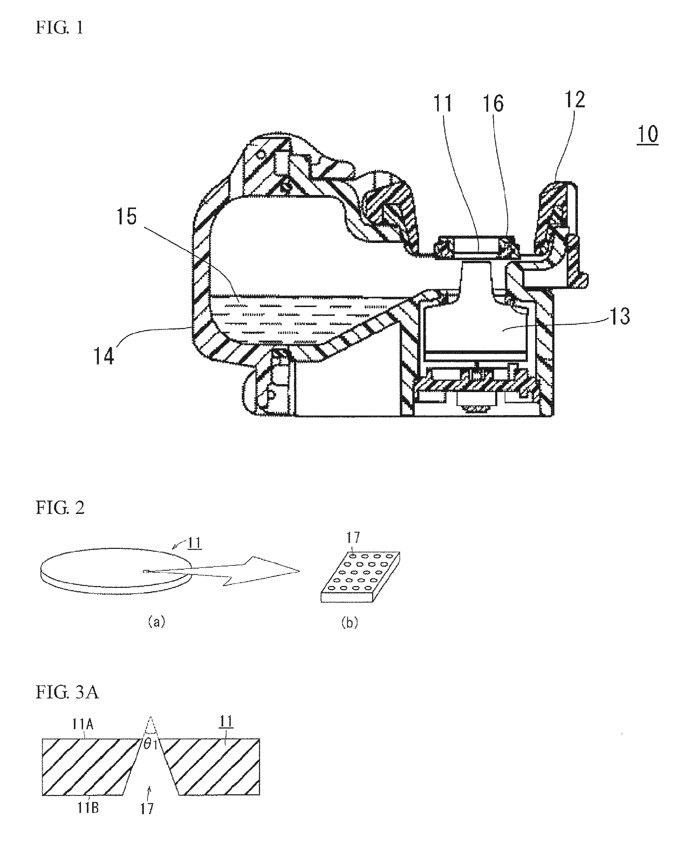

FIG. 1 is a schematic cross-sectional view illustrating a structure of an inhalator according to an embodiment of the present invention.
[FIG. 2]
FIGS. 2(a) and 2(b) are a perspective view and a partially enlarged view illustrating the appearance of a mesh according to the present embodiment.
[FIG. 3A]
FIG. 3A is a schematic partially cross-sectional view illustrating a cross-section of the mesh, which passes an outlet of a through hole and is parallel to a discharging direction.
[FIG. 3B]

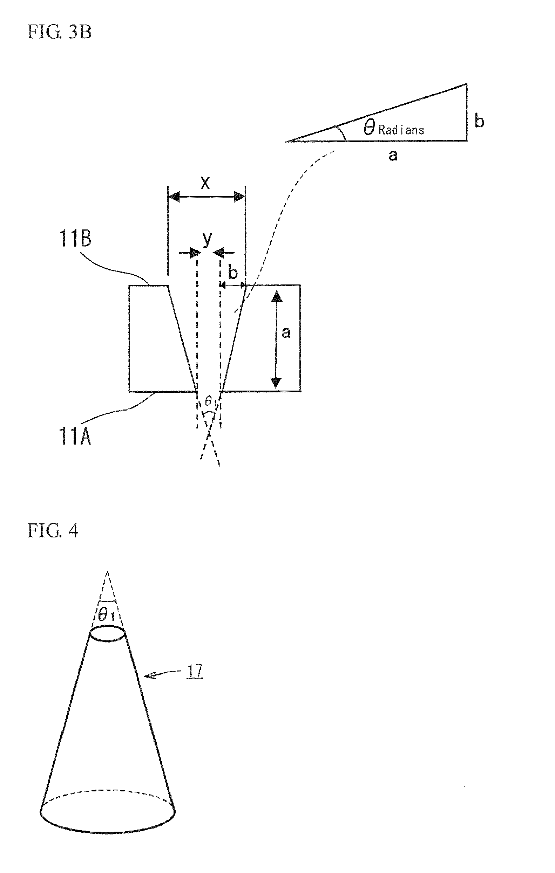

FIG. 3B is a schematic partially cross-sectional view illustrating how to obtain a taper angle $\theta_1$.
[FIG. 4]
FIG. 4 is a schematic perspective view illustrating a shape of a through hole.
[FIG. 5]

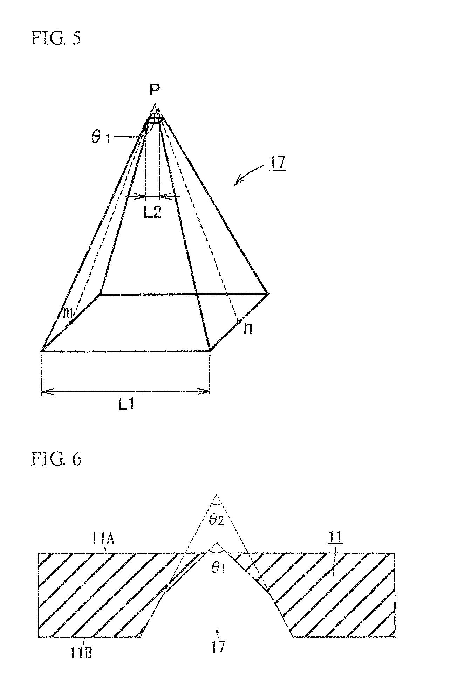

FIG. 5 is a schematic perspective view illustrating a shape of a through hole.
[FIG. 6]
FIG. 6 is a schematic partially cross-sectional view illustrating a cross-section of the mesh, which passes an outlet of a through hole and is parallel to a discharging direction.
[FIG. 7]

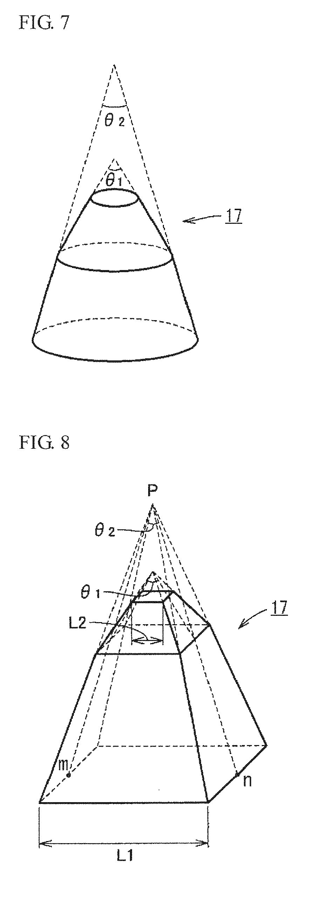

FIG. 7 is a schematic perspective view illustrating a shape of a through hole.
[FIG. 8]
FIG. 8 is a schematic perspective view illustrating a shape of a through hole.
[FIG. 9]

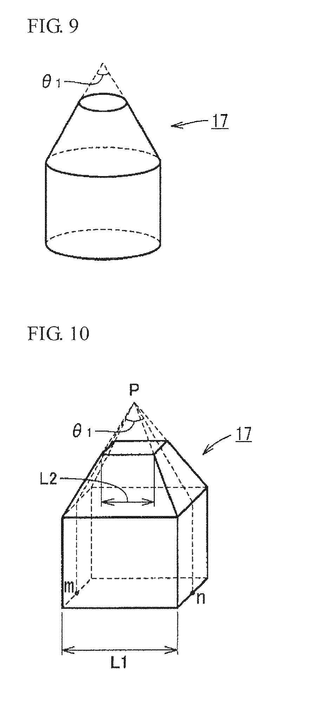

FIG. 9 is a schematic perspective view illustrating a shape of a through hole.
[FIG. 10]
FIG. 10 is a schematic perspective view illustrating a shape of a through hole.
[FIG. 11]

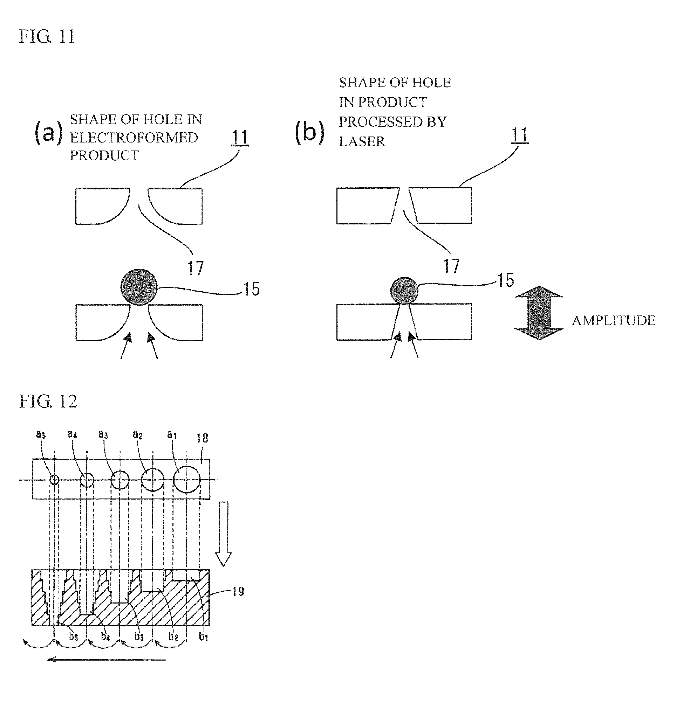

FIGS. 11(a) and 11(b) are views illustrating the superiority of a laser-processed product, based on the difference between products respectively processed by different processing-methods.
[FIG. 12]
FIG. 12 is a view illustrating a method of producing a nebulizer mesh according to the present invention.
[FIG. 13]

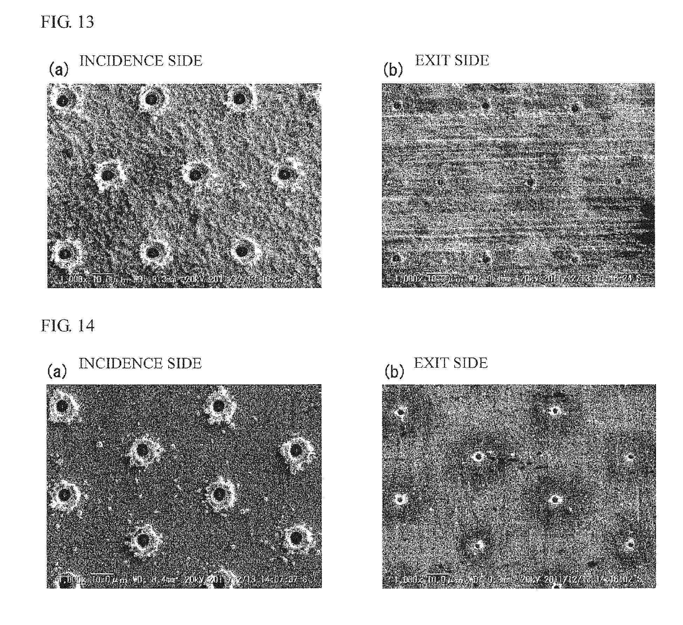

FIGS. 13(a) and 13(b) are views respectively illustrating images of through holes taken by a scanning electron microscope (a magnification of 1000) (Example 1).
[FIG. 14]
FIGS. 14(a) and 14(b) are views respectively illustrating images of through holes taken by a scanning electron microscope (a magnification of 1000) (Example 2).
[FIG. 15]

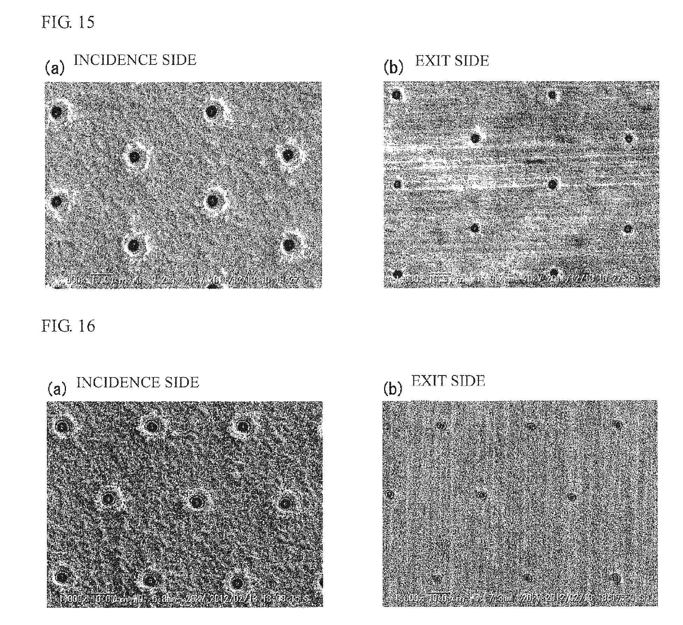

FIGS. 15(a) and 15(b) are views respectively illustrating images of through holes taken by a scanning electron microscope (a magnification of 1000) (Example 3).
[FIG. 16]
FIGS. 16(a) and 16(b) are views respectively illustrating images of through holes taken by a scanning electron microscope (a magnification of 1000) (Example 4).

[FIG. 17]

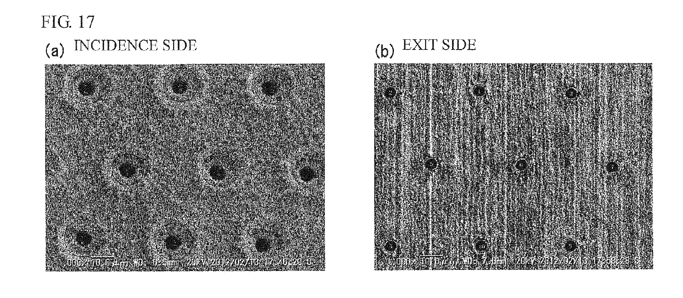

FIGS. 17(a) and 17(b) are views respectively illustrating images of through holes taken by a scanning electron microscope (a magnification of 1000) (Example 5).

[Mode for Carrying Out the Invention]

Hereinafter, the best mode for carrying out the present invention is described.

FIG. 1 illustrates a configuration of the best nebulizer for carrying out the present invention. However, the present invention is not limited to the configuration at all. FIG. 1 is a schematic cross-sectional view illustrating a configuration in the case of using a nebulizer mesh according to the present invention (hereinafter also referred to simply as a mesh) in an atomizing inhalator (nebulizer) 10 of the ultrasonic vibration type. The inhalator 10 is configured such that a mesh 11 having plural fine through holes, as illustrated in FIG. 2, is fixed in a casing 12; that the top surface of a vibrator 13 is pressed against the bottom surface of the mesh 11; and that an end portion of the vibrator 13 is contactable with liquid 15 stored in a thank 14. The mesh 11 is held by a mesh support 16.

The mesh 11 used in the inhalator 10 is constituted by a large number of sufficiently fine through holes 17, as illustrated in FIG 2, in order to convert the liquid 15 into fine particles (i.e., atomize). FIG. 2 is a perspective view (a) and a partially enlarged view (b) illustrating the appearance of the mesh 11. As illustrated in FIG. 2(a), the mesh 11 has a plate-like outer shape. In addition, as illustrated in FIG. 2(b), the mesh 11 has the plural through holes 17.

When the vibrator 13 is caused to vibrate up and down, the mesh 11 pressed by the mesh support 16 against the vibrator 13 with an appropriate force by the mesh support 16 resonates due to microvibrations of the vibrator 13. When the mesh 11 resonates, a negative pressure is generated between the mesh 11 and the vibrator 13. Thus, the liquid 15 stored in the tank 14 is sucked up to the top surface of the vibrator 13. The liquid 15 sucked up to between the mesh 11 and the vibrator 13 passes through the fine through holes 17 due to the vibration of the mesh 11. Thus, the atomized liquid 15 is discharged into outside-air.

FIG. 3 is a schematic partially cross-sectional view illustrating a cross-section of the mesh 11, which passes an outlet of a through hole 17 and is parallel to a discharging direction. FIGS. 4 and 5 are schematic perspective views each illustrating a shape of a through hole 17. As illustrated in FIG. 3, the through hole 17 in the mesh 11 according to the present invention has a tapered shape whose width at the side of the outlet surface 11A in an atomizing direction of the mesh is narrower than the width at the side of the inlet surface 11B of the mesh.

According to the present invention, the expression "through hole has a tapered shape" means that the outlet surface side hole diameter of the through hole is smaller than the inlet-surface-side hole diameter of the through hole. A form of a through hole is not particularly limited as long as it has a tapered shape. Examples thereof include a conically shape, a pyramid like shape, a halfway-narrowed conically shape, a halfway-narrowed pyramid like shape, step-wise shape and the like.

The taper angle $\theta_1$ (see FIG. 3A) is calculated by measuring the outlet surface side hole diameter and the inlet surface side hole diameter with the scanning electron microscope. More specifically, the taper angle $\theta_1$ is calculated as follows. The average value of the hole areas of ten holes which are optionally extracted from an SEM (scanning electron microscope) image of a produced mesh nozzle at the side of the inlet surface 11B (corresponding to the larger hole diameter) and at the side of the outlet surface 11A (corresponding to the smaller hole diameter) are calculated. The taper angle is calculated (see FIG. 3B) according to the following formulae based on the calculated hole diameter (incidence diameter) corresponding to the inlet surface 11B and the calculated hole diameter (exit diameter) corresponding to the outlet surface 11 A.

$$(\text{incidence diameter})_x \cdot (\text{exit diameter})_y = \sqrt{(2 \times (hole - area) / \pi)}$$

$$\theta_{\text{Radians}} = \tan^{-1}(b/a)$$

$$\theta_{\text{Degrees}} = \theta_{\text{Radians}} \times 180/\pi$$

$$(\text{taper angle}) = 2\theta_{\text{Degrees}}$$

$$b = (\text{incidence diameter})_x - (\text{exit diameter})_y$$

$$\text{(taper angle)}\theta_1 = 2\times\tan^{-1}[\{\text{(incidence diameter x)-(exit diameter y)}\}/\text{(base-material thickness)}_a]\times180/\pi$$

The taper angle $\theta_1$ of the through hole 17 in the outlet surface 11A ranges from 3 degrees to 30 degrees and preferably ranges from 3 degrees to 20 degrees and more preferably ranges from 3 degrees to 10 degrees. The average particle diameter (nebulized particle diameter) of particles nebulized from the nebulizer mesh depends on the volume of the through hole 17 in the mesh. Thus, if the taper angle is large even when the diameter of the through hole 17 in the outlet surface 11A is the same, the volume of extruded liquid to be displayed increases. Consequently, the nebulized-particle diameter increases.

The nebulizer mesh according to the present invention can reduce the volume of the extruded liquid, which is nebulized therefrom, by reducing the taper angle $\theta_1$ of the through hole 17 at the side of the outlet surface 11A to a small value, e.g., 3 degrees to 30 degrees. The reduction of the extruded liquid to be nebulized enables increase of the outlet surface side diameter of each particle having the optimum particle diameter (3 $\mu$m to 6 $\mu$m) for allowing particles atomized from liquid such as a drug solution to reach target organs such as alveoli. This facilitates the processing of the through holes.

In the nebulizer mesh according to the present invention, the taper angle $\theta_1$ ranges from 3 degrees to 30 degrees and is small. Thus, the discharge resistance at the time of nebulizing the liquid can be reduced. The clogging of the through hole with the liquid can be reduced. In addition, a sufficient amount of nebulized liquid can be attained. In addition, since the discharge resistance can be reduced, the thickness of the mesh can be increased. Consequently, sufficient-stiffness can be ensured.

If the taper angle $\theta_1$ exceeds 30 degrees, an amount of liquid nebulized per unit time is increased. However, the number of nebulized particles increases. Thus, it is difficult to set the nebulized-particle diameter at a value at which the drug solution reaches the alveoli. In addition, the discharge resistance at the time of nebulizing the liquid increases. Thus, the clogging of the through holes with the liquid is likely to occur. This may lead to reduction of the amount of nebulized liquid. If the taper angle $\theta_1$ is less than 3 degrees, the nebulized particles tend to return to the liquid due to surface tension. This may lead to reduction of the amount of nebulized liquid.

The through hole 17 can be, e.g., conically-shaped, as illustrated in FIG. 4. Consequently, the nebulized-particle diameter of particles nebulized from the through hole 17 can be decreased.

The through hole 17 may also be shaped like a pyramid, as illustrated in FIG. 5. In this connection, the through hole 17 illustrated in FIG. 5 has an inlet diameter L1 and an outlet diameter L2. The taper angle $\theta_1$ at the side of the outlet surface 11A is the angle mPn where m and n designate the midpoints of opposite sides of the bottom surface and P denotes a vertex.

FIG. 5 illustrates a case where both of an inlet and an outlet are square-shaped. However, if the shapes of the inlet and the outlet are general polygons, each of L1 and L2 is defined as the distance between two points at which a straight-line passing through the centroid of the polygon and the midpoint of a side of the polygon, which is closest to the centroid, intersects with sides of the polygon, respectively. The taper angle $\theta_1$ is defined as the angle mPn where m and n designates the two points at which the above straight line intersects with the sides of the polygon, and P denotes the vertex.

FIG. 6 is a schematic partially cross-sectional view illustrating a cross-section which passes through the outlet of the through hole 17 of the mesh 11 and is parallel to a discharging direction. FIGS. 7 to 10 are schematic perspective views illustrating the shapes of the through holes.

In FIG. 3, it is described that the case where the wall surface of the through hole 17 in the mesh 11 is linearly-shaped from the inlet to the outlet. However, the through hole 17 may have a cross-sectional shape halfway-narrowed such that as illustrated in FIG. 6, a part of the through hole 17, which is located at the side of the outlet surface 11A, has a first taper angle $\theta_1$, and that another part of the through hole 17, which is located at the side of the inlet surface 11B of the mesh nozzle, has a second taper angle $\theta_2$ smaller than the first taper angle $\theta_1$.

The halfway-narrowed shape of the through hole 17 may be a halfway-narrowed conically shape, as illustrated in FIG. 7. Namely, both of the part having the first taper angle $\theta_1$, and the part having the second taper angle $\theta_2$ may be conically-shaped.

The halfway-narrowed shape of the through hole 17 may be a pyramid-like shape halfway-narrowed, as illustrated in FIG. 8. That is, both of the part having the first taper angle $\theta_1$, and the part having the second taper angle $\theta_2$ may be shaped like a pyramid.

In addition, the halfway-narrowed shape of the through hole 17 may be such that as illustrated in FIG. 9, the part having the second taper angle is cylindrically-shaped, while the part having the first taper angle is conically-shaped. The halfway-narrowed shape of the through hole 17 may be such that as illustrated in FIG. 10, the part having the second taper angle is cylindrically-shaped, while the part having the first taper angle is conically-shaped.

Although the first taper angle $\theta_1$ of each of the through holes 17 illustrated in FIGS. 5 to 10 can appropriately be chosen, preferably, the first taper angle $\theta_1$ ranges from 3 degrees to 30 degrees. In this connection, the inlet diameter and the outlet diameter of the pyramid-like part of each of the through holes 17 illustrated in FIGS. 8 and 10 are determined by the inlet diameter L1 and the outlet diameter L2 in FIGS. 8 and 10, similarly to the case of the through hole illustrated in FIG. 5.

The taper angles $\theta_1$ and $\theta_2$ of the through hole 17 illustrated in FIG. 8 and the taper angles $\theta_1$ of the through hole 17 illustrated in FIG. 10 are determined by the angle mPn where m and n designate the midpoints of opposite sides of the bottom surface and P denotes the vertex.

The nebulizer mesh according to the present invention contains platinum as a main component of the material thereof. The expression "contains platinum as a main component" indicates that the content of platinum contained in the material constituting the nebulizer mesh is equal to or more than 50 % by mass. Thus, the content of platinum contained in the material condtituting the nebulizer mesh is equal to or more than 50 % by mass. It is preferable that the content is 70 % by mass or higher and it is more preferable that the content is 90 % by mass or higher.

For example, precious metals such as gold, silver, iridium, rhodium, ruthenium, palladium, and valve metals such as titanium, zirconium, hafnium, niobium, tantalum, zinc, tungsten, and bismuth can be cited as components other than platinum, which are contained in the material constituting the nebulizer mesh.

The nebulizer mesh according to the present invention employs, as the main component, platinum whose specific gravity is high in comparison with those of other metals. The specific gravity of platinum is 21.45 g/cm$^3$. For example, the specific gravity of nickel is 8.902 g/cm$^3$ and the specific gravity of palladium is 12.02 g/cm$^3$, which has hitherto been used as the material of the nebulizer mesh. The kinetic energy K of the mesh is represented by K = (1/2) x mv$^2$ wherein m designates a mass and v represents a velocity. The larger the mass becomes, the higher the kinetic energy does. Therefore, when the liquid is nebulized, high kinetic energy can be obtained using, as the main component, platinum which is high in specific gravity in comparison with other metals. Even when the taper angle is set to be low, high-viscosity liquid and high-specific-gravity liquid can be nebulized. The influence of the type of liquid is low. Thus, it is estimated to enable stable nebulizing and increase of an amount of nebulized liquid.

The nebulizer mesh according to the present invention has high bio-safety in view of metal allergy and carcinogenicity with using platinum as the main component. In addition, stiffness is secured and the thickness of the nebulizer mesh can be reduced. The liquid resistance is reduced by decreasing the thickness of the mesh. Thus, the nebulizing of the liquid is more facilitated. Consequently, an amount of nebulized liquid can be increased

Preferably, the thickness of the nebulizer mesh is equal to or thicker than 10 $\mu$m. More preferably, the thickness of the mesh is equal to or thicker than 15 $\mu$m. Still more preferably, the thickness of the mesh is equal to or thicker than 20 $\mu$m. In addition, preferably, the thickness of the mesh is equal to or thinner than 300 $\mu$m. More preferably, the thickness of the mesh is equal to or thinner than 100 $\mu$m. Still more preferably, the thickness of the mesh is equal to or thinner than 50 $\mu$m. Sufficient strength can be ensured by setting the thickness of the nebulizer mesh to be equal to or thicker than 10 $\mu$m. The density of platinum is increased by setting the thickness of the nebulizer mesh to be equal to or thinner than 300 $\mu$m. Thus, the inertia mass of the mesh can be prevented from being increased. The response of the mesh to ultrasonic vibrations can be suppressed from being degraded.

The number of the through holes 17 for a nebulizer according to the present invention is not limited to a specific value, and can appropriately be set according to the type and the purpose of the liquid 15. Usually, preferably, the number of the through holes 17 is equal to or more than 100. More preferably, the number of the through holes 17 is equal to or more than 1000. Still more preferably, the number of the through holes 17 is equal to or more than 10000.

Preferably, the outlet surface side diameter of the through hole 17 in the nebulizer mesh according to the present invention is equal to or larger than 1.5 $\mu$m. More preferably, the outlet surface side diameter of the through hole 17 in the mesh is equal to or larger than 2 $\mu$m. Still more preferably, the outlet surface side diameter of the through hole 17 in the mesh is equal to or larger than 2.5 $\mu$m. In addition, the outlet surface side diameter of the through hole 17 in the mesh is equal to or smaller than 6 $\mu$m. More preferably, the outlet surface side diameter of the through hole 17 in the mesh is equal to or smaller than 5 $\mu$m. Still more preferably, the outlet surface side diameter of the through hole 17 in the mesh is equal to or smaller than 4 $\mu$m.

Preferably, the nebulized-particle diameter of particles nebulized by the nebulizer mesh according to the present invention ranges from 3 to 6 $\mu$m. More preferably, the nebulized-particle diameter ranges from 3.5 $\mu$m to 5.5 $\mu$m. Still more preferably, the nebulized-particle diameter ranges from 4.0 $\mu$m to 5.0 $\mu$m, Effects, such as the effects of treatment, due to the liquid can be more enhanced. For example, it can be set to be the optimum particle diameter of particles atomized from a drug solution that reaches target organs such as alveoli.

The average particle diameter of particles nebulized from the nebulizer mesh is measured by the following method. The produced mesh nozzle is installed in a nebulizer having a horn vibrator. Next, a physiological saline solution is supplied as the liquid to a region in which the vibrator and the mesh nozzle are contacted with each other to be atomized. The average particle diameter of particles to be nebulized is measured by a laser light diffraction method with using a particle diameter measurement apparatus. More specifically, the average particle diameter can be measured by a method which will be described in detail in the following description of Examples.

The nebulizer mesh according to the present invention can be produced by performing laser processing as a hole processing technique. FIG. 11 is a view illustrating products processed by an electroforming method and a laser method, respectively, for comparing these methods with each other. As illustrated in FIG. 11, the mesh is vibrated up and down by an ultrasonic device. When the amplitude of vibration is increased, an amount of the liquid 15 extruded from the through hole 17 is also increased. Thus, the nebulized-particle diameter is increased.

The electroformed product is such that the taper angle is large. Thus, the shape of the electroformed product cannot be controlled. In order to decrease the nebulized-particle diameter, it is necessary to make the diameter of the through hole 17 extremely small. Thus, in the case of using the electroforming method, it is difficult to control the diameter and the taper angle. However, in the case of using laser processing, the diameter and the taper angle can be controlled by changing conditions.

On the other hand, the product processed by the laser method can be shaped such that the taper angle ranges from 3 degrees to 30 degrees. Thus, the laser method has an advantage in that it is unnecessary to reduce the diameter. Since the reduction of the diameter is unnecessary, the clogging of the through hole with the liquid such as a drug solution can be prevented.

Lasers used for the laser method are, e.g., the following lasers classified by wavelength, that is, an ultraviolet laser, an infrared laser, a visible-light laser, an X-ray laser, and the like. An optimum conditions such as kinds of lasers can appropriately be selected according to a processing material and a processing purpose. From the viewpoint of absorption wavelengths of platinum, an ultraviolet laser is suited to the processing of the nebulizer mesh according to the present invention, which contains platinum as the main component. The ultraviolet laser is laser light whose wavelength is less than 400 nm which is an ultraviolet region. Lasers are also classified by a laser gain medium. Thus, solid state lasers such as a YAG laser, a rare earth doped YAG laser, a ruby laser, and a titanium sapphire laser; liquid lasers such as a dye laser using an organic dye as a laser gain medium; and a gas laser such as an excimer laser are cited as the laser used for the laser method.

For example, a fixed optical system (light collection optical system), a scanning optical system, and an imaging optical system (mask optical system) are cited as the types of optical systems used for the laser method. From the viewpoint of positional accuracy and reproducibility, the imaging optical system is preferable.

According to a production method for the nebulizer mesh according to the present invention, a flat-plate-like member containing platinum as the main component is irradiated with laser light to form plural cross sectionally tapered shaped through holes. More specifically, the following method is cited as the production method for the mesh. Namely, the method uses a processing mask 18 provided with circular windows $a_1$, ..., $a_5$ having diameters respectively corresponding to several stages (5 stages in the example illustrated in FIG. 12) of taper systems of micro-holes, as illustrated in FIG. 12. For ease of description, five windows provided in the single mask 18 are illustrated. However, actually, five masks each provided with an associated one of the windows respectively corresponding to the different stages are prepared. Thus, the mesh is produced by replacing masks with another one of the masks.

Among the windows $a_1$, ..., $a_5$, the window $a_1$ corresponds to the inlet surface side of the micro-hole, the window $a_5$ corresponds to the outlet surface side of the micro-hole, and the windows $a_2$, ..., $a_4$ correspond to halfway locations in the micro-hole. First, the flat plat like member 19 having predetermined thickness and size and having platinum as a main component of the material thereof is irradiated with laser-light utilizing the window $a_1$ of the processing mask 18. The window $a_1$ narrows an irradiation diameter for irradiating laser-light. Thus, a hole $b_1$ whose diameter is equal to the diameter of the window $a_1$ is formed in the flat plate like member 19. Laser-light irradiation is continued until the hole $b_1$ has a predetermined depth.

Then, laser light is irradiated from the window $a_1$ so as to form a hole $b_2$ which is deeper than the hole $b_1$. Similarly, laser-light is irradiated utilizing the windows $a_3$ and $a_4$. Thus, holes $b_3$ and $b_4$ are dug, respectively. Then, laser light is irradiated from the window $a_5$ to form a hole $b_5$. Consequently, a hole is formed, which passes through the flat plate like member 19 from the inlet surface side to the outlet surface side. Thus, micro-holes having a cross sectionally tapered shape are formed in a manner that the diameter thereof is reduced in stages according to holes $b_1$ to $b_5$ in order,.

Examples of processing-dimensions are shown. The thickness of the flat plate like member 19 is 20 $\mu$m. The diameter of the hole $b_1$ ranges from 3.5 $\mu$m to 6.5 $\mu$m. The diameter of the hole $b_5$ is 2.5 $\mu$m. With such a production method, a mesh member can be provided, which has micro-holes with high accuracy and employs platinum as the main component of the material thereof.

Although the micro-holes are formed by dividing a production processes into five stages in the case of the specific example of the above manufacturing method, the number of the stages may be either larger or smaller than five and can appropriately be chosen. In addition, micro-holes having various cross-sectional shapes can be formed according to the diameter and the number of windows of a processing mask 18.

The nebulizer mesh according to the present invention is formed by using a material which contains platinum as a main component and which is safe to human bodies. Thus, the mesh can be used in either medical apparatuses such as an inhalator illustrated in FIG. 1, or cosmetology apparatuses such as a facial care device.

Especially, in the case of the inhalator, a distance therefrom to an affected area which the liquid can reach is largely changed according to the nebulized-particle diameter. Thus, effects of the liquid on the affected area are changed. The nebulized particle diameter largely depends on the taper angle and the shape of the mesh holes. However, according to the present invention, mesh holes can be obtained, each of which has an optimum cross-sectional shape for nebulizing liquid.

In addition, even if the frequency of the vibrator is the same as before, the taper angle and the shape of the mesh hole can be changed according to an affected area or intended-use. Accordingly, even the same inhalator can be used for plural affected-areas only by replacing the mesh with another mesh.

According to the present invention, the term "liquid" to which the nebulizer mesh according to the present invention is applied includes, e.g., organic substances, inorganic substances, solutions of the organic or organic substances, inorganic substances, and ceramic substances, and slurry liquid products thereof. Preferably, the solutions of organic substances/inorganic substances are used as the liquid. For example, a drug solution and a cosmetic material are cited as the liquid.

[Examples]

Hereinafter, the present invention is described with reference to Examples. However, the present invention is not limited thereto. In this connection, evaluation methods used in the examples are described below.

[Examples 1 to 7]

Each nebulizer mesh was manufactured by forming 10,000 through holes in a metal base material with 20 μm thickness (formed of a rolled platinum foil) with laser-light, whose wavelength was 355 nm, in processing-stages of the number described in Table 1, by using a solid-state ultraviolet laser oscillator (product name: Q301-HD (manufactured by JDS Uniphase Corporation)).

[Comparative Example 1]

A nebulizer mesh was formed similarly to the case of Example 1 except that a Pd (75%)-Ag (25%) (manufactured by Nilaco Corporation) was used as the material of the metal base material.

[Comparative Example 2]

A platinum nebulizer mesh with 20 μm thickness was formed by peeling a resist off a copper plate after forming a plated platinum layer having a large number (10,000) of holes by electroforming on the copper plate on which the resist was formed (Please see JP-A-9-323054).

The taper angle and the nebulized particle diameter of the manufactured nebulizer mesh were measured by a method described below.

(Taper Angle)

The taper angle θ1 was calculated as follows. The average value of the hole areas of ten holes which were optionally extracted from an SEM (scanning electron microscope) image of a produced mesh nozzle, at the side of the inlet surface (corresponding to a larger hole-diameter) and at the side of the outlet surface (corresponding to the smaller hole-diameter), are calculated. The diameters of equivalent perfect circles calculated from the average values of the hole areas were set as the hole diameters respectively. The taper angle was calculated according to the following formulae from the calculated hole diameter (incidence diameter x) corresponding to the inlet surface and the calculated hole diameter (exit diameter y) corresponding to the outlet surface.

$$\text{(incidence diameter x)} \cdot \text{(exit diameter y)} = \sqrt{2 \times (hole - area) / \pi}$$

$$\theta_{Radians} = \tan^{-1}(b/a)$$

$$\theta_{Degrees} = \theta_{Radians} \times 180/\pi$$

$$(\text{taper angle}) = 2\theta_{\text{Degrees}}$$

$$b = (\text{incidence diameter x})-(\text{exit diameter y})$$

$$(\text{taper angle})\theta_1 = 2\times\tan^{-1}[\{(\text{incidence diameter x})-(\text{exit diameter y})\}/(\text{base material thickness a})]\times 180/\pi$$

[Measurement of Nebulized Particle Diameter]

The produced mesh nozzle was installed in the nebulizer (product name: NE-U22 (manufactured by OMRON Corporation) having a horn vibrator. Next, a physiological saline solution was supplied as the liquid to a region in which the vibrator and the mesh nozzle were contacted with each other. Then, the physiological solution was atomized. The average particle diameter (i.e., the nebulized particle diameter) of particles to be nebulized was measured by a laser light diffraction method using a particle diameter measurement apparatus (product name: HELOS/BR-Multi (manufactured by SYMPATEC Corporation)).

Results are described in Table 1. In addition, FIGS. 13 to 17 illustrate data representing images taken at the incidence side (inlet-surface side) and the exit side (outlet surface side) by a scanning electron microscope. In Table 1, if the rate of the number of non-through holes to the number of all through holes is equal to or less than 0.1%, it is indicated that hole-processing defects are "absent". On the other hand, if the rate of the number of non-through holes to the number of all through holes exceeds 0.1%, it is indicated that hole-processing defects are "present". If the intensity of laser light due to nebulized liquid is less than 99.7% at the measurement of the nebulized-particle diameter, it is indicated that nebulizing-defect is "absent". If the intensity of laser light due to the nebulized liquid is equal to or higher than 99.7% at the measurement of the nebulized particle diameter, it is indicated that nebulizing-defect is "present".

Table 1

| | Base Material | Processing Method | The Number of Processing Stages | Inlet Surface Side Hole Area (μm²) | Outlet Surface Side Hole Area (μm²) | Taper Angle (degrees) | Hole Processing Defect | Nebulized-Particle Diameter (μm) | Presence/Absence of Nebulizing-Defect |
|---|---|---|---|---|---|---|---|---|---|
| Example 1 | Plated Platinum Foil | Laser Processing | 1 | 23.495 | 7.7108 | 6.7 | Absent | 4.8 | Absent |
| Example 2 | Rolled Platinum Foil | Laser Processing | 1 | 28.3053 | 4.1397 | 10.6 | Absent | 3 | Absent |
| Example 3 | Plated Platinum Foil | Laser Processing | 1 | 23.8177 | 11.7419 | 4.7 | Absent | 8.9 | Absent |
| Example 4 | Plated Platinum Foil | Laser Processing | 2 | 21.5935 | 6.8397 | 6.6 | Absent | 5.7 | Absent |
| Example 5 | Plated Platinum Foil | Laser Processing | 2 | 190.9231 | 23.606 | 28.4 | Absent | 21.3 | Absent |
| Example 6 | Plated Platinum Foil | Laser Processing | 1 | 26.053 | 16.5732 | 3.3 | Absent | 5.9 | Absent |
| Example 7 | Rolled Platinum Foil | Laser Processing | 1 | 20.4543 | 4.2149 | 7.9 | Absent | 3.8 | Absent |
| Comparative Example 1 | Rolled Pd-Ag Foil | Laser Processing | 1 | 21.256 | 4.242 | 8.2 | Absent | - | Present |
| Comparative Example 2 | Platinum | Electro-forming | - | 480.862 | 5.668 | 57.8 | Present | 4.9 | Absent |

As described in Table 1, in the case of the meshes for a nebulizer according to Examples 1 to 7, all holes penetrated through the mesh and no hole processing defects such as variation in hole-diameter were detected. On the other hand, in the case of Comparative Example 1 using a rolled Pd-Ag foil as the base material, all holes penetrated through the mesh, and no hole processing defects such as variation in hole-diameter were detected. However, since a nebulizing-defect was detected, no nebulized particle diameter could be measured. In addition, in the case of the nebulizer mesh according to Comparative Example 2, which was manufactured by electro-forming, there was variation in the hole diameter. It was confirmed that about 1% of all holes didn't penetrate through the mesh. A hole-processing defect was found.

It was known from these results that the nebulizer mesh according to the present invention can nebulize particles whose particle diameters range from 3 $\mu$m to 20 $\mu$m without causing hole-processing defects and nebulizing-defects.

[Industrial Applicability]

The nebulizer mesh according to the present invention can be applied to medical apparatuses such as an inhalator, and to cosmetic apparatuses such as a facial care device.

Although the present invention has been described in detail with reference to the specific embodiments, it is apparent to those skilled in the art that the present invention can be changed or modified in various manners without departing from the spirit and scope of the present invention.

This application claims priority from Japanese Patent Application No. 2012-074060 filed on March 28, 2012, and Japanese Patent Application No. 2012-235493 filed on October 25, 2012, which are hereby incorporated by reference herein in its entirety.

[Description of Reference Numerals and Signs]

10    inhalator

11    mesh

12    casing

13    vibrator

14    tank

15    liquid

16    mesh support

17    through hole

18    mask

19    flat plate like member

**Claims**

1.  A mesh for use in a nebulizer for atomizing and nebulizing liquid,
    wherein the mesh is used to nebulize the liquid, and configured to have a plurality of through holes;
    wherein each of the plurality of through holes has a tapered shape whose width is gradually narrowed towards an outlet surface side of the mesh;
    wherein platinum is used as a main component of a material of the mesh, **characterized in that** each of the plurality of through holes has a taper angle at the outlet surface side that ranges from 3 degrees to 30 degrees.

2.  The mesh for use in a nebulizer according to claim 1, wherein a thickness of the mesh is equal to or larger than 10 $\mu$m.

3. The mesh for use in a nebulizer according to claim 1 or 2, wherein an outlet surface side diameter of each of the through holes is equal to or larger than 1.5 µm.

4. A method for producing a mesh for use in a nebulizer according to claim 1, wherein a flat-plat-like member which has platinum as a main component is irradiated with laser light in order to form a plurality of through holes, each of which being cross-sectionally tapered shaped, and wherein each of the plurality of through holes has a taper angle at the outlet surface side that ranges from 3 degrees to 30 degrees.

5. The method for producing a mesh for use in a nebulizer according to claim 4, wherein the laser light is ultraviolet laser light.


**Patentansprüche**

1. Sieb zur Verwendung in einem Zerstäuber zum Vernebeln und Zerstäuben von Flüssigkeit,
   wobei das Sieb verwendet wird, um die Flüssigkeit zu zerstäuben, und so konfiguriert ist, dass es eine Mehrzahl von Durchgangslöchern aufweist;
   wobei jedes der Mehrzahl von Durchgangslöchern eine verjüngte Form aufweist, deren Breite hin zu einer Auslassflächenseite des Siebs schrittweite verengt ist;
   wobei Platin als eine Hauptkomponente eines Materials des Siebs verwendet wird, **dadurch gekennzeichnet, dass** jedes der Mehrzahl von Durchgangslöchern einen Kegelwinkel auf der Auslassflächenseite aufweist, der im Bereich von 3 Grad bis 30 Grad liegt.

2. Sieb zur Verwendung in einem Zerstäuber nach Anspruch 1, wobei eine Dicke des Siebs größer gleich 10 µm ist.

3. Sieb zur Verwendung in einem Zerstäuber nach Anspruch 1 oder 2, wobei ein Auslassflächenseitendurchmesser jedes der Durchgangslöcher größer gleich 1,5 µm ist.

4. Verfahren zum Herstellen eines Siebs zur Verwendung in einem Zerstäuber nach Anspruch 1, wobei ein flachzopfähnliches Element, das Platin als eine Hauptkomponente aufweist, mit einem Laserlicht bestrahlt wird, um eine Mehrzahl von Durchgangslöchern zu bilden, wobei jedes davon im Querschnitt eine verjüngte Form aufweist, und wobei jedes der Mehrzahl von Durchgangslöchern einen Kegelwinkel auf der Auslassflächenseite aufweist, der im Bereich von 3 Grad bis 30 Grad liegt.

5. Verfahren zum Herstellen eines Siebs zu Verwendung in einem Zerstäuber nach Anspruch 4, wobei das Laserlicht ultraviolettes Laserlicht ist.


**Revendications**

1. Crible pour utilisation dans un nébuliseur pour atomiser et nébuliser un liquide,
   où le crible est utilisé pour nébuliser le liquide, et configuré de manière à avoir une pluralité de trous traversants ;
   dans lequel chacun de la pluralité de trous traversants a une forme conique dont la largeur est progressivement rétrécie vers un côté de surface de sortie du crible ;
   dans lequel du platine est utilisé en tant que composant principal d'un matériau du crible, **caractérisé en ce que** chacun de la pluralité de trous traversants a un angle de cône au côté de surface de sortie qui est dans la plage de 3 degrés à 30 degrés.

2. Crible pour utilisation dans un nébuliseur selon la revendication 1, dans lequel l'épaisseur du crible est égale ou supérieur à 10 µm.

3. Crible pour utilisation dans un nébuliseur selon la revendication 1 ou 2, dans lequel le diamètre côté surface de sortie de chacun des trous traversants est égal ou supérieur à 1,5 µm.

4. Procédé de production d'un crible pour utilisation dans un nébuliseur selon la revendication 1, dans lequel un élément de type plaque plate qui comporte du platine en tant que composant principal est irradié avec une lumière laser afin de former une pluralité de trous traversants, dont chacun est de forme conique en section transversale, et dans lequel chacun de la pluralité de trous traversants a un angle de cône au côté surface de sortie qui est dans la plage de 3 degrés à 30 degrés.

5. Procédé de production d'un crible pour utilisation dans un nébuliseur selon la revendication 4, dans lequel la lumière laser est une lumière laser ultraviolette.

FIG. 1

FIG. 2

(a)                 (b)

FIG. 3A

FIG. 3B

FIG. 4

FIG. 5

FIG. 6

FIG. 7

FIG. 8

FIG. 9

FIG. 10

FIG. 11

(a) SHAPE OF HOLE IN ELECTROFORMED PRODUCT

11

17

15

(b) SHAPE OF HOLE IN PRODUCT PROCESSED BY LASER

11

17

15

AMPLITUDE

FIG. 12

$a_5$  $a_4$  $a_3$  $a_2$  $a_1$  18

19

$b_5$  $b_4$  $b_3$  $b_2$  $b_1$

FIG. 13

(a) INCIDENCE SIDE

(b) EXIT SIDE

FIG. 14

(a) INCIDENCE SIDE

(b) EXIT SIDE

FIG. 15

(a) INCIDENCE SIDE

(b) EXIT SIDE

FIG. 16

(a) INCIDENCE SIDE

(b) EXIT SIDE

FIG. 17

(a) INCIDENCE SIDE

(b) EXIT SIDE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2790014 B
- JP 9323054 A
- JP 7001172 A
- JP 2006297226 A
- JP 2008289903 A
- JP 6320084 A
- JP 2012074060 A
- JP 2012235493 A

**Non-patent literature cited in the description**

- *OMRON TECHNICS,* 2002, vol. 42, 171-175
- *The Transactions E of the Institute of Electrical Engineers of Japan,* 1997, vol. 117 (1), 15-19